# FASCICULE DE BREVET EUROPEEN

(11) **EP 1 038 551 B1**
(45) Date de publication et mention de la délivrance du brevet: **17.08.2005**
(21) Numéro de dépôt: 00400748.0
(22) Date de dépôt: 17.03.2000
(51) Int. Cl.: A61N 7/02, A61B 8/00

(54) **Milieu de couplage pour ultrasons de puissance**
Kopplungsmedium für therapeutischen Ultraschall
Coupling agent for therapeutic ultrasound

(30) Priorité: 25.03.1999 FR 9903738
(43) Date de publication de la demande: 27.09.2000
(73) Titulaire: TECHNOMED MEDICAL SYSTEMS, 69120 Vaulx-en-Velin (FR)
(72) Inventeur: Godo, Joseph, 77100 Meaux (FR); Blanc, Emmanuel, 69230 Saint Genis Laval (FR)
(74) Mandataire: Cabinet Hirsch

(56) Documents cités:
- EP-A- 0 344 773
- EP-A- 0 420 758
- US-A- 4 886 068
- US-A- 5 666 954
- US-A- 5 720 286

## Description

La présente invention concerne le domaine des ultrasons, et plus précisément le domaine des ultrasons de puissance utilisés à des fins thérapeutiques, et notamment pour créer des lésions dans des tissus.

Il est connu d'utiliser les ultrasons à des fins de diagnostic, dans des dispositifs d'échographie; ces dispositifs sont généralement constitués d'une simple sonde, qui est appliquée contre le patient. Afin d'améliorer le couplage des ultrasons, il est connu d'utiliser un gel de couplage, entre la sonde et la peau du patient. Ce gel est appliqué en couche mince sur la peau du patient, avant le début de l'examen.

JP-A-2 92343 décrit un produit de couplage pour l'imagerie ultrasonique, formé d'une matrice en matériau poreux, tel du polyuréthanne, contenant un gel aqueux tel de la polyvinylpyrrolidone (PVP).

On utilise aussi des ultrasons à des fins thérapeutiques, pour la destruction de concrétions, principalement par effet mécanique (onde de choc), ou pour créer des lésions dans des tissus, principalement par effet thermique. Les appareils utilisés dans ce cas comprennent un transducteur, qui est disposé dans une enceinte souple ou rigide, remplie d'un liquide de couplage, le plus souvent de l'eau. L'enceinte est appliquée contre le patient, au voisinage de la zone à traiter. On peut le cas échéant utiliser un gel de couplage entre l'enceinte et les tissus du patient. La distance de propagation dans le liquide de couplage est plus importante que dans le cas d'un gel de couplage pour l'échographie.

On peut citer à titre d'exemple d'appareils de traitement par ultrasons les dispositifs d'hyperthermie endo-cavitaire des documents FR 91 02 620, FR 93 09 158, FR 96 08 096, FR 94 01 304 ou FR 94 06 539; ces dispositifs sont adaptés au traitement de la prostate par voie endo-rectale, et comprennent une sonde de thérapie focalisée, qui peut produire un échauffement des tissus au point focal, et provoquer une nécrose des tissus se trouvant au point focal.

L'invention s'applique notamment au domaine du traitement thérapeutique des tissus par ultrasons focalisés, et plus particulièrement le domaine de la destruction de tissus à l'intérieur d'un organisme en provoquant de hautes températures à l'aide d'ultrasons focalisés. Dans le domaine général des ultrasons focalisés, comme l'homme du métier le sait, on distingue différents types de traitements: le traitement le plus ancien est le traitement par lithotripsie, qui s'applique à la destruction de corps durs; ce type de traitement utilise des ondes de chocs, i.e. des impulsions courtes et de forte puissance. Il a ensuite été proposé de traiter les tissus mous par hyperthermie, en chauffant les tissus à des températures peu élevées, i.e. inférieures à 45°C. L'hyperthermie implique l'envoi vers les tissus à traiter d'ultrasons sous forme d'impulsions longues et de puissance plus faible. Enfin, sont maintenant proposés des traitements de tissus mous par ultrasons focalisés de haute intensité, généralement appelés traitements HIFU (acronyme de l'anglais High Intensity Focused Ultrasounds). Les traitements HIFU consistent à chauffer des tissus à des températures élevées, typiquement supérieures à 45°C. Les traitements par ultrasons focalisés de haute intensité (HIFU) représentent un moyen efficace de créer des lésions de nécrose de coagulation dans les tissus biologiques en vue de traiter les tumeurs localisées.

Au delà d'un certain seuil de puissance, les ultrasons créent dans les milieux au sein desquels ils se propagent des phénomènes de cavitation. Outre les effets mécaniques de la cavitation, les bulles créées par la cavitation sont un obstacle à la propagation des ultrasons. Le phénomène de cavitation est d'autant plus gênant que la puissance des ultrasons est importante. Ces phénomènes de cavitation ne se produisent pas en échographie, l'intensité des ultrasons utilisés étant inférieure au seuil de cavitation des agents de couplage. En outre, dans les applications échographiques, l'épaisseur d'agent de couplage est telle que l'absorption des ultrasons par le gel de couplage est secondaire.

Dans le cas de dispositifs de thérapie comprenant en outre une surveillance par échographie, comme dans les demandes précitées, la présence de bulles provoquées par la cavitation peut en outre être gênante pour l'imagerie ou simplement pour la mesure de distance, même si les bulles de cavitation ne posent pas de problèmes pour le passage des ultrasons de thérapie. Notamment, lors de la mesure de distance par échographie en mode A, les bulles de cavitation créent des échos parasites qui perturbent et faussent la mesure.

US-A-5 445 813, au nom de Schneider et autres, propose une suspension injectable à base de microbulles, utilisée comme agent de contraste pour l'échographie. Cette suspension agit comme un réflecteur des ultrasons; elle est échogène.

Cette utilisation va à l'encontre des buts de la présente invention, dans la mesure où elle tend à augmenter l'absorption par des ultrasons de faible puissance, au lieu, comme le propose l'invention de diminuer la cavitation provoquée par les ultrasons de forte puissance.

A. Rajulu et autres, Ultrasonic studies in solutions of polyvinylpyrrolidone, Acustica (1991), vol. 75 no 3, pages 213-6 évalue les propriétés des solutions aqueuses de PVP pour déterminer les variations de la compressibilité adiabatique en fonction de la température, de la viscosité, et du rapport molaire entre la polyvinylpyrrolidone et le solvant.

K. Rao et autres, Acoustical parameters of polyvinylpyrrolidone solutions, Acta polym. (1989), vol. 40 no 12, pages 743-6 démontre la présence d'un complexe à liaisons en forme de H pour des solutions aqueuses de PVP à diverses concentrations, à partir de l'étude de la vitesse de propagation des ultrasons, de la viscosité, et de l'indice de réfraction.

S. Kato et autres, Ultrasonic absorption of polymer solutions. XIII. Ultrasonic absorption in aqueous polyvinylpyrrolidinone solutions, Nippon Kagaku Kaishi (1974), vol. 10, décrit le comportement en absorption de solutions aqueuses de PVP entre 5 et 130 MHz et entre 10 et 40°C.

Ces documents ne décrivent pas et ne suggèrent pas non plus l'utilisation de solutions aqueuses de PVP ou d'autre polymère hydrophile comme milieu de couplage pour des ultrasons de forte puissance.

US-A-5 078 149 décrit un dispositif de couplage ultrasonique, constitué d'un récipient contenant un gel polymère ; le dispositif de couplage est destiné à être disposé entre une sonde ultrasonique d'échographie et le patient à observer. Un des gels polymères proposés dans ce document est une solution de PVP réticulée dans trois dimensions par irradiation. Ce document ne mentionne que des applications à l'échographie, et ne mentionne pas le problème de la cavitation, ni l'application au traitement par ultrasons de puissance.

Par ailleurs, les transducteurs de puissance doivent être refroidis; le milieu de couplage en contact avec ceux-ci doit donc être pompable (liquide), ce qui exclut les milieux visqueux.

Document US-A-5 666 954 décrit une sonde endorectale thérapeutique comprenant un transducteur de thérapie apte à émettre des ultrasons, disposé dans une enceinte remplie d'un milieu de couplage.

Il existe donc un besoin d'un milieu de couplage liquide qui soit approprié à la transmission des ultrasons aux fréquences et aux énergies utilisées habituellement pour les traitements de thérapie ultrasonique, et qui permette d'éviter autant que possible les phénomènes de cavitation. De préférence, un tel milieu de couplage devrait présenter une absorption aussi faible que possible dans la plage de fréquence des ultrasons utilisés.

Ce problème se pose notamment pour les appareils d'hyperthermie, mais aussi pour les autres appareils de traitement par ultrasons de forte puissance.

L'invention propose une solution à ce problème.

Plus précisément, l'invention propose un milieu de couplage pour ultrasons de puissance comprenant une solution aqueuse liquide d'un polymère hydrophile.

Selon un mode de réalisation, le polymère hydrophile est la polyvinylpyrrolidone.

Selon un mode de réalisation, le poids moléculaire du polymère hydrophile est compris entre 10 000 et 100 000.

Selon un mode de réalisation, la quantité de polymère est comprise entre 10 et 50 g/l.

L'invention a aussi pour objet un appareil de thérapie par ultrasons, comprenant un transducteur de thérapie apte à émettre des ultrasons de puissance, disposé dans une enceinte remplie d'un milieu de couplage selon l'invention.

Selon un mode de réalisation, l'appareil comprend un générateur d'ultrasons couplé au transducteur, le générateur étant couplé au transducteur de sorte que celui-ci émet des ultrasons dans une plage de fréquence entre 1 et 5 MHz, de préférence entre 2 et 3 MHz.

Selon un mode de réalisation, l'appareil comprend en outre un transducteur d'imagerie.

Selon un mode de réalisation, l'appareil comprend en outre un dispositif de mesure de la distance à la cible par ultrasons selon la technique d'échographie en mode A.

Selon un mode de réalisation, l'appareil comprend en outre des moyens de recirculation dudit milieu de couplage.

D'autres caractéristiques et avantages de l'invention apparaîtront à la lecture de la description qui suit de modes de réalisation de l'invention, donnée à titre d'exemple uniquement.

Le milieu de couplage selon l'invention est adapté aux ultrasons de puissance. On entend, dans le cadre de la présente invention, par "ultrasons de puissance" tous ultrasons capables de créer des dommages dans les tissus par destruction ou nécrose, en particulier des dommages thermiques ou par effet de cavitation, ces dommages étant le but de ces ultrasons (par opposition aux ultrasons d'échographie qui parfois créent des dommages involontairement). Les termes d'ultrasons de puissance et ultrasons de thérapie sont, dans la demande, la même signification. Typiquement, les ultrasons de puissance ou de thérapie présentent une densité d'énergie supérieure à 1000 W/cm².

Le milieu de couplage selon l'invention est une solution aqueuse liquide d'un polymère hydrophile. Cette solution est liquide, donc pompable, et donc peut servir de fluide de refroidissement du transducteur, et/ou de la sonde, notamment dans le cas d'une sonde endo-rectale, et/ou des tissus avoisinant et/ou des tissus à traiter.

On entend, dans le cadre de la présente invention, par "polymère hydrophile" toute substance de poids moléculaire élevé, (par exemple supérieur à 300) ayant une affinité suffisante pour l'eau pour s'y dissoudre ou y former un gel. Des exemples de tels polymères sont : polyvinylpyrrolidone, polymères acryliques, poly(alcool vinylique), dérivés cellulosiques (tels que alkylcellulose, hydroxypropylcellulose, hydroxyméthylcellulose, hydroxypropylméthylcellulose, carboxyméthylcellulose), gélatine, gommes (guar, agar-agar, etc.), polyéthylèneoxyde, etc.. Des mélanges de polymères sont aussi appropriés. Le poids moléculaire est par exemple compris entre 10 000 et 100 000, préférence entre 30 000 et 75 000.

Le polymère hydrophile préféré est la polyvinylpyrrolidone (PVP). La PVP utilisée dans le cadre de la présente invention est par exemple la Plasdone, notamment K29-32 (poids moléculaire d'environ 58 000).

La concentration en polymère dans le milieu aqueux peut varier dans de larges mesures, déterminées par l'homme de l'art. En général, la quantité de polymère est comprise entre 10 et 50 g/l. Utilisé en cette quantité, le polymère hydrophile ne conduit pas à une solution gélifiée, par opposition à l'art antérieur. La viscosité de la solution de l'invention est sensiblement celle de l'eau (qui est de 1 cst (centistoke) à 20°C) . La viscosité de la solution selon l'invention est comprise entre 1 et 2 cst, de préférence entre 1,2 et 1,6 cst, à 20°C. A titre d'exemple, cette viscosité est de 1,3 cst pour une solution de PVP à une concentration de 10g/l.

Les caractéristiques d'absorption acoustique, de la solution selon l'invention sont sensiblement les mêmes que celles de l'eau. L'atténuation de la solution selon l'invention est identique à celle de l'eau, par exemple pour les solutions de PVP à une concentration de 10 et 20g/l.

La faible absorption acoustique, ou la faible atténuation acoustique du milieu de couplage de l'invention permet que la majeure partie des ultrasons de puissance soit effectivement transmise vers la zone à traiter.

En termes de cavitation, l'invention permet aussi une amélioration sensible par rapport à l'utilisation connue de l'eau comme milieu de couplage.

Le milieu de couplage de l'invention permet de la sorte de diminuer les effets de la cavitation, sans pour autant augmenter l'absorption des ultrasons. Il assure une bonne transmission des ultrasons de thérapie; en outre, il assure que le dispositif d'imagerie, s'il existe, fournit une image nette de la zone à traiter, et que le dispositif de mesure, notamment par échographie en mode A, fournit une distance correcte de la cible.

Le milieu de couplage de l'invention est un milieu à base d'eau; il est donc facile à préparer et à utiliser, et ne nécessite pas de précautions particulières d'usage. Il présente l'avantage d'être relativement fluidité, et dans le cas d'un transducteur contenu dans une enveloppe souple, s'adapte ainsi facilement à la morphologie de la zone traitée.

Le milieu de couplage selon l'invention peut contenir les additifs classiques, tels que stabilisants, agents anti-bactériens (par exemple l'amphorisine) et autres.

Le milieu de couplage de l'invention présente en outre une impédance acoustique voisine de celle de l'eau.

La vitesse de propagation dans le milieu de l'invention est aussi sensiblement égale à celle de l'eau. A titre d'exemple les vitesses de propagation dans la solution selon l'invention sont égales, pour des solutions de PVP à une concentration de 10 et 20g/l, à celles dans l'eau augmentées respectivement de 3 et 5%.

L'identité des vitesses de propagation et des impédances permet d'utiliser le milieu de couplage de l'invention dans les dispositifs de thérapie existants, sans qu'il soit nécessaire de les modifier. En outre, l'utilisation d'un milieu de couplage ayant de telles caractéristiques de propagation évite la défocalisation et les réflexions sur les interfaces. On obtient un milieu de couplage présentant sensiblement les caractéristiques de propagation des tissus du corps humain, ce qui assure une bonne focalisation, et une bonne transmission, notamment aux interfaces.

L'invention propose en outre un appareil de traitement par ultrasons, comprenant un transducteur de thérapie disposé dans une enceinte contenant un tel milieu de couplage. L'enceinte peut être une enceinte rigide, souple ou partiellement souple. L'appareil peut comprendre un générateur d'ultrasons, apte à générer des ultrasons de thérapie, par exemple des ultrasons dans une plage de fréquence de le générateur émettant des ultrasons dans une plage de fréquence de 1 à 5 MHz, dé préférence de 2 à 3 MHz.

De préférence, l'appareil de l'invention présente aussi un transducteur d'imagerie, qui fournit aussi une image de la zone traitée. De préférence, l'appareil de l'invention présente un dispositif de mesure par échographie en mode A à partir du transducteur de thérapie, ledit dispositif étant un élément d'émission-réception intégré au transducteur (envoi impulsion et mesure du temps de retour de l'impulsion i.e. mesure du temps de l'écho). L'utilisation d'un milieu de couplage selon l'invention permet d'assurer qu'un tel dispositif fournit aussi une image nette et/ou une mesure de la distance à la cible fiable.

L'exemple suivant illustre l'invention sans la limiter.

### Exemple

On prépare des solutions de Plasdone K29-32 à des concentrations de 10, 20 et 50g/l. On obtient des solutions fluides liquides.

Le milieu de couplage de l'invention présente des caractéristiques d'absorption très proches de celles de l'eau.

Ce milieu de couplage est introduit dans un appareil de thérapie du type de celui commercialisé par la demanderesse sous le nom de "Ablatherm". Outre une influence nulle au niveau de l'effet thérapeutique, ce milieu de couplage évite les échos parasites lors de la mesure de la distance à cible par la technique de l'échographie en mode A.

Bien entendu, la présente invention n'est pas limitée aux exemples et modes de réalisation décrits et représentés, mais elle est susceptible de nombreuses variantes accessibles à l'homme de l'art.

## Revendications

1. Un appareil de thérapie par ultrasons, comprenant un transducteur de thérapie apte à émettre des ultrasons de puissance, disposé dans une enceinte remplie d'un milieu de couplage, **caractérisé en ce que** ledit milieu de couplage comprenant une solution aqueuse liquide pompable d'un polymère hydrophile.

2. L'appareil selon la revendication 1, **caractérisé en ce que**, dans le milieu de couplage, le polymère hydrophile est la polyvinylpyrrolidone.

3. L'appareil selon la revendication 1 ou 2, **caractérisé en ce que**, dans le milieu de couplage, le poids moléculaire du polymère hydrophile est compris entre 10 000 et 100 000.

4. L'appareil selon la revendication 1, 2 ou 3, **caractérisé en ce que**, dans le milieu de couplage, la quantité de polymère est comprise entre 10 et 50 g/l.

5. L'appareil selon l'une quelconque des revendications 1 à 4, **caractérisé en ce qu'**il comprend un générateur d'ultrasons couplé au transducteur, le générateur étant couplé au transducteur de sorte que celui-ci émet des ultrasons dans une plage de fréquence entre 1 et 5 MHz, de préférence entre 2 et 3 MHz.

6. L'appareil selon l'une quelconque des revendications 1 à 5, **caractérisé en ce qu'**il comprend en outre un transducteur d'imagerie.

7. L'appareil selon l'une quelconque des revendications 1 à 6, **caractérisé en ce qu'**il comprend en outre un dispositif de mesure de la distance à la cible par ultrasons selon la technique d'échographie en mode A.

8. L'appareil selon l'une quelconque des revendications 1 à 7, **caractérisé en ce qu'**il comprend en outre des moyens de recirculation dudit milieu de couplage.

9. Utilisation à titre de milieu de couplage pour ultrasons de puissance d'une solution aqueuse liquide pompable d'un polymère hydrophile.

10. Utilisation selon la revendication 9, **caractérisée en ce que** ladite solution est une solution de polyvinylpyrrolidone, ayant un poids moléculaire compris entre 10 000 et 100 000, en une quantité de polymère comprise entre 10 et 50 g/l.

## Patentansprüche

1. Eine Ultraschall-Therapievorrichtung umfassend einen Therapie-Ultraschallwandler, der geeignet ist um Ultraschall hoher Leistung zu emittieren, angeordnet in einer Einfassung, die mit einem Kopplungsmedium gefüllt ist, **dadurch gekennzeichnet, dass** dieses Kopplungsmedium eine wässrige, flüssige, pumpfähige Lösung eines hydrophilen Polymers umfasst.

2. Vorrichtung gemäß Anspruch 1, **dadurch gekennzeichnet, dass** das hydrophile Polymer in dem Kopplungsmedium Polyvinylpyrrolidon ist.

3. Vorrichtung gemäß Anspruch 1 oder 2, **dadurch gekennzeichnet, dass** das Molekulargewicht des hydrophilen Polymers in dem Kopplungsmedium zwischen 10.000 und 100.000 liegt.

4. Vorrichtung gemäß Anspruch 1, 2 oder 3, **dadurch gekennzeichnet, dass** die Menge an Polymer in dem Kopplungsmedium zwischen 10 und 50 g/l liegt.

5. Vorrichtung gemäß einem der Ansprüche 1 bis 4, **dadurch gekennzeichnet, dass** sie einen Ultraschallgenerator umfasst, der an den Ultraschallwandler gekoppelt ist, wobei der Generator so an den Wandler gekoppelt ist, dass dieser Ultraschallwellen in einem Frequenzbereich zwischen 1 und 5 MHz, bevorzugt zwischen 2 und 3 MHz, emittiert.

6. Vorrichtung gemäß einem der Ansprüche 1 bis 5, **dadurch gekennzeichnet, dass** sie ferner einen Wandler zur Bildgebung umfasst.

7. Vorrichtung gemäß einem der Ansprüche 1 bis 6, **dadurch gekennzeichnet, dass** sie ferner eine Einrichtung zum Messen des Abstandes zum Ziel durch Ultraschall nach der Echographie-Technik im Modus A umfasst.

8. Vorrichtung gemäß einem der Ansprüche 1 bis 7, **dadurch gekennzeichnet, dass** sie ferner Mittel zur Rezirkulation des genannten Kopplungsmediums umfasst.

9. Verwendung einer wässrigen, flüssigen, pumpfähigen Lösung eines hydrophilen Polymers als Kopplungsmedium für Ultraschall hoher Leistungen.

10. Verwendung gemäß Anspruch 9, **dadurch gekennzeichnet, dass** die Lösung eine Lösung von Polyvinylpyrrolidon ist, die ein Molekulargewicht zwischen 10.000 und 100.000 aufweist, mit einer zwischen 10 und 50 g/l liegenden Menge an Polymer.

## Claims

1. An ultrasound therapy apparatus, comprising a therapy transducer adapted to transmit high-power ultrasound, arranged in an enclosure filled with a coupling medium, **characterized in that** said coupling medium comprises a pumpable liquid aqueous solution of a hydrophilic polymer.

2. The apparatus according to claim 1, **characterized in that**, in the coupling medium, the hydrophilic polymer is polyvinylpyrrolidone.

3. The apparatus according to claim 1 or 2, **characterized in that**, in the coupling medium, the molecular weight of the hydrophilic polymer is comprised between 10,000 and 100,000.

4. The apparatus according to claim 1, 2 or 3, **characterized in that**, in the coupling medium, the amount of polymer is comprised between 10 and 50 g/l.

5. The apparatus according to any one of claims 1 to 4, **characterized in that** it comprises an ultrasound generator coupled to the said transducer, the generator being coupled to the transducer whereby the latter transmits ultrasound in a frequency range between 1 and 5 MHz, preferably between 2 and 3 MHz.

6. The apparatus according to any one of claims 1 to 5, **characterized in that** it further comprises an imaging transducer.

7. The apparatus according to any one of claims 1 to 6, **characterized in that** it further comprises a device for measuring the distance to the target using ultrasound employing an A-mode ultrasound technique.

8. The apparatus according to any one of claims 1 to 7, **characterized in that** it further comprises means for recirculating said coupling medium.

9. Use of a pumpable liquid aqueous solution of a hydrophilic polymer as a coupling medium for high-power ultrasound.

10. Use according to claim 9, where the solution is a solution of polyvinylpyrrolidone, having a molecular weight comprised between 10,000 and 100,000, in an amount of polymer comprised between 10 and 50 g/l.
